# EUROPEAN PATENT SPECIFICATION

(11) **EP 4 087 529 B1**
(45) Date of publication and mention of the grant of the patent: **09.09.2026**
(21) Application number: 21703977.5
(22) Date of filing: 07.01.2021
(51) Int. Cl.: A61H 35/04, A61M 15/08, B65D 83/30, A61H 33/02, B05B 1/34

(54) **DISPENSER**
SPENDER
DISTRIBUTEUR

(30) Priority: 08.01.2020 IT 202000000160; 04.03.2020 IT 202000001048 U
(43) Date of publication of application: 16.11.2022
(73) Proprietor: Mezzoli, Giorgio, 48022 Lugo (RA) (IT); Rani, Maria, 48022 Lugo (RA) (IT)
(72) Inventor: Mezzoli, Giorgio, 48022 Lugo (RA) (IT); Rani, Maria, 48022 Lugo (RA) (IT)
(74) Representative: Roncuzzi, Davide
(86) International application number: PCT/IB2021/050094
(87) International publication number: WO 2021/140461

(56) References cited:
- WO-A1-2012/079097
- US-A1- 2014 121 592

## Description

The present invention concerns a dispenser of liquids for medical uses. In particular, the present invention concerns a liquid dispenser comprising a membrane body provided with at least one nozzle in apical position. In further detail, the present invention concerns a gravity feed liquid dispenser provided with at least two nozzles designed to effectively spray human or animal body cavities.

### DESCRIPTION OF THE STATE OF THE ART

The sector of dispensers of solutions, including medical solutions, comprises dispensers that can be used for washing the inside of human and animal body cavities with said solutions, including the nasal cavities, the oral cavity, the vaginal cavity and the outer ear canal, without minimizing the multiple uses of said devices.

A large part of said dispensers have been conceived and designed for washing the nasal cavities.

The nasal cavities form the uppermost part of the respiratory tract and are separated by the nasal septum, which is an osteo-cartilaginous wall coated by mucosa. Each cavity is split into areas having different anatomical shapes according to their functions. A brief description of these areas is provided below.

The area of the nasal vestibule is the outermost part of the nose. The nasal vestibule is medially delimited by a rigid component, the nasal septum, and delimited laterally and at the top by an elastic component, the wings of the nose. The structures that compose the nasal vestibule give it the shape of an oval or elliptical funnel, the coronal section area of which tapers from the outside towards the inside. The nasal vestibule has the function of conveying the air inhaled towards the nasal cavity.

The valve area: the nasal valve or ostium internum is the transition point between the nasal vestibule and the nasal cavity. The upper part of the valve area is called nasal valve, and consists of the junction between the rigid component, namely the nasal septum, arranged medially, and the elastic component of the wing of the nose, arranged supero-laterally. It is the elastic structure that regulates the direction and flow rate of the air currents, during both inhalation and exhalation.

The area of the nasal conchae: each nasal cavity communicates at the front with the nasal vestibule through the nasal valve and at the rear with the nasopharynx through the choanal area; medially it is delimited by the nasal septum and laterally by the lateral wall from which bony convolutions called nasal turbinates, which are covered by a highly vascularized and innervated mucosa. There are generally 3 nasal turbinates in each nasal cavity - lower, middle and upper; they are positioned between the nasal septum and the lateral wall of the nasal cavity and delimit irregular spaces called lower meatus, middle meatus and upper meatus respectively. In the upper and middle part of the nasal cavity, below the cribriform plate, the air space of which is reduced to a width of 1-2 mm, is the olfactory region, located above the middle nasal turbinate, between the nasal septum and the lateral wall. The olfactory region is covered by olfactory epithelium, present only in this region. Each nasal cavity communicates, through small ducts, with the respective paranasal sinuses: the maxillary sinuses, the frontal sinuses and the anterior ethmoid sinuses are connected to the middle meatus; the rear ethmoid sinuses and the sphenoid sinuses open into the upper meatus. The choanal area is the terminal part of the nasal cavity through which each nasal cavity communicates with the nasopharyngeal area. The nasopharyngeal area is very important since it communicates with the cavity of the middle ear through the Eustachian tube and due to the presence of the adenoid tissue.

The nasal mucosa consists of a pseudo-stratified epithelium in which the majority of the cells are ciliated cells, caliciform cells and basal cells; the epithelium rests on a basal membrane and the latter on the plate in which capillaries, arterio-venous anastomoses, seromucous glands and cavernous sinusoids are present. The cilia are immersed in a "sol" aqueous fluid, produced by the anterior serous glands, by the seromucous glands, by the transudate of the vessels and by the condensed water of the exhaled air; on the surface there is a very thin layer of sticky mucus, a sort of "gel" produced by the caliciform cells and by the seromucous glands. The pH of the nasal mucosa is approximately 5.5-6.5. The cilia move rhythmically at a frequency of 16/sec i.e., 1000/min, determining the movement of the mucous surface layer towards the oropharynx which, in normal conditions, takes place in approximately 12 min (the transport speed is extremely variable from 3 to 25 mm/min); in children the mucous is carried towards the adenoids, increasing contact with the immune system. The mucociliary system serves to humidify, warm and cleanse the inhaled air. The body temperature is ideal for the ciliary movement. The temperature influences the mucociliary system: between 32°C and 40°C the ciliary movement reaches maximum functionality and remains constant. The mucociliary system constitutes a natural barrier which has the function of protecting against pathogens and cleansing the inhaled substances.

In normal conditions the nasal cavities perform the function of conditioning the inhaled air, warming it, humidifying it and cleansing it, and the exhaled air, reabsorbing heat and humidity. The turbinates enable these functions by giving the respiratory air a turbulent motion so as to increase the surface inside the nasal cavity in contact with the air.

On the other hand, washing of the nasal cavities is performed by causing the washing solution to flow inside one of the two nasal cavities, keeping the head tilted forward. The solution will flow out from the other nasal cavity after passing through the nasopharynx, having mechanically removed any pathological secretions present. The pressure at which the solution is introduced into the nasal cavity must be limited in order not to damage the mucosa that covers the entire nasal cavity. Throughout the nasal washing the user must keep his/her head tilted forward and breathe through the mouth which must remain open. This prevents even small quantities of the solution getting into the lower airways causing coughing or breathing difficulty which, in the most serious cases, can lead to bronchospasm. From the above description, it is evident that nasal washes are not suitable for very young patients, who tend not to tolerate invasive treatments, especially when the required application times are long. For this reason, in babies and non-collaborative patients it is preferable to administer solutions, including medical solutions, inside the nasal cavities in the form of spray, aerosol or micronized douche. In order for these applications to be effective, the washing liquid, in its path inside the nasal cavity, must be able to spray all the anatomical structures it encounters on its way passing through the nasopharynx and discharging the used washing liquid through the other nasal cavity. It is useful to specify that the pressure value at which the washing liquid is supplied must be sufficient to remove any pathological secretions inside the nasal cavities while not damaging the anatomical structures that compose the mucosa covering the entire nasal cavity, so as not to negatively interfere with the protective action of the mucociliary system.

Of the known devices, the most effective ones are those that implement the teachings contained in the European Patent EP 0814747 of the applicants, where the delivery pressure of the liquid is determined exclusively by gravity. With particular reference to said patent EP '747, a given quantity of the liquid used to perform the nasal washing, or washing of any other body cavity, is contained in a container, a bag or a tank, having given capacity. Said container is connected by a flexible tube to a dispenser, which is shaped to be inserted alternatively inside the two nostrils; the container must be kept above the reclined head of the user throughout the washing operation. Therefore, the pressure of the liquid during the washing is always proportional to the difference in level between the free surface of the liquid in the container and the height of the dispenser inserted in the nostrils. Considering that the length of the tube connecting the container to the dispenser is approximately 60 cm, the washing pressure corresponding to the level difference will be approximately 0.055-0.060 atmospheres, therefore very low and compatible with the mucociliary system.

Again according to the patent EP '747, the dispenser has a dome-shaped body constructed symmetrically with respect to two median planes that cross in a central axis. Of these median planes, one is frontal and the other is sagittal, and to each of them corresponds an axis of symmetry of given extension for the sections transversal to the central axis of said dome-shaped body. Said geometric conformation is compatible with that of the nostrils and, therefore, the dispenser can be pushed into the nasal vestibule and nasal valve to the point where the outer wall of the dome-shaped body adheres univocally and appropriately to the inner walls of said anatomical structures. The dispenser has three channels, the axes of which lie on the median plane to which the major axis of the dome-shaped body corresponds. Each channel ends in a conical-shaped apical portion which opens at the apex of the dome-shaped body with a hole, so that the holes corresponding to the three channels are aligned on the major axis of the dispenser.

The dispenser cited in the patent EP '747 has the drawback that when the quantity of liquid in the tank decreases, the water column that pushes the liquid downwards, and therefore the driving force acting on the liquid, drops. In particular, when the liquid level drops, the three jets coming out of the three holes tend to join, so that said jet is no longer able to spray all the anatomical structures that compose the nasal cavity safeguarding the integrity of the mucociliary system.

The above drawback has pushed the applicants to consider even more advanced embodiments of the dispensers, which guarantee that all the areas inside the nasal cavity are sprayed by the washing liquid even when the level of the liquid contained in the container/bag/tank decreases and, likewise, the pressure of the liquid pertaining to the dispenser, determined by gravity.

Of the known device,document US2014121592A1 discloses a dispenser comprising all the technical features set out in the preamble of claim 1.

### SUMMARY OF THE PRESENT INVENTION

The present invention is directed to a dispenser according to claim 1. Additional features and embodiments of the invention are defined in the dependent claims.

### SUMMARY OF THE DISCLOSURE

The present invention concerns a liquid dispenser for medical uses. In particular, the present invention concerns a liquid dispenser comprising a membrane body provided with at least one nozzle in apical position. In further detail, the present invention concerns a gravity feed liquid dispenser provided with at least two nozzles designed to effectively spray human or animal body cavities.

Aim of the present invention is to provide a gravity feed dispenser which is without the drawbacks described above and which therefore allows liquid solutions to be dispensed in the form of single jets oriented constantly towards the lower, middle and upper meatus of the nasal cavities even when the pressure of the liquid fed to the dispenser drops due to the progressive emptying of the container containing the liquid, resulting from reduction in the level of the liquid in the container and, therefore, the force of gravity that pushes the liquid through the dispenser nozzles. According to the present invention a simple inexpensive dispenser is provided which allows liquid solutions to be dispensed in the form of single jets towards the lower, middle and upper meatus of the nasal cavities, where said jets remain separate even when the pressure of the liquid inside the container drops due to the progressive emptying of said container, said emptying causing progressive reduction of the force of gravity acting on the washing liquid.

The problems illustrated above are solved by the present invention according to at least one of the following claims. According to some embodiments of the present invention a liquid dispenser for medical uses is provided in which a first membrane body is shaped symmetrically with respect to a first frontal median plane and to a second sagittal median plane intersected in a central axis and is delimited at the top by a dome which is axially delimited by a base portion and by a top of given shape; a second body being carried by said first body inside said dome in a position concentric with said axis; said second body having a lower hollow portion concentric with said axis and an upper portion engaged by a first and a second duct ending in said dome, with a first nozzle and a second nozzle respectively; each of said first and second nozzles being arranged between said first frontal median plane and said second sagittal median plane.

According to one embodiment, said first and second nozzles are arranged at the same radial distance from said central axis on planes parallel to said second sagittal median plane.

According to one embodiment said first and second ducts have respective longitudinal axes parallel to said central axis and arranged at substantially identical distances from said central axis.

According to the present invention said longitudinal axes of said first and second ducts lie on said first plane or are arranged on a plane which is centred on the central axis and is inclined with respect to said first frontal median plane and second sagittal median plane.

According to one embodiment said top is faceted and has a central flat portion transversal to said axis and four faces that extend from said flat portion around said axis; two first faces of said four faces being of equal shape and extension, in addition to being arranged symmetrically with respect to said median plane, and two faces of said four faces being of equal shape and extension, in addition to being arranged symmetrically with respect to said second median plane.

According to one embodiment, each of said first and second nozzles are obtained in each of said second faces at the back of a respective outer edge.

According to one embodiment said upper portion is engaged by a third duct concentric with said central axis; said third duct ending with a third nozzle obtained centrally in said central flat portion.

According to one embodiment, said third duct extends inside said lower portion; said first, second and third ducts extend from said base portion to said top.

According to one embodiment, said upper portion has a cylindrical portion which is concentric with said axis and is contained inside said lower portion to define with the latter an axial cylindrical guide for an end portion of a cylindrical supply duct of said liquid.

According to one embodiment, said cylindrical portion has an outer shell provided with two longitudinal grooves coaxial with the said first and second ducts to define, in use, a supply channel for each said first and second ducts when said end portion of said cylindrical duct couples with said cylindrical guide.

According to one embodiment, said lower portion is hollow and is provided with a central chamber concentric to said axis.

According to one embodiment, said lower portion is hollow and is provided with a common central chamber supplying said first, second and third ducts.

According to some embodiments of the present invention, a gravity feed liquid dispenser for medical uses is provided in which a first membrane body has at the top a dome axially delimited by a base portion and by a top of given shape provided with a central axis positioned at the intersection of a first frontal median plane and a second sagittal median plane; a second body being carried internally by said first body; said second body having a lower hollow portion concentric with said central axis and an upper portion engaged by a first duct and by a second duct, both ending in said dome, with a first and a second nozzle respectively; said first and second ducts having respective terminal sections which end in said first and second nozzles with outlet direction which, moving away from said first plane, is distant point by point from said second plane by a length greater than or equal to the distance of the corresponding said first and second nozzles from said second plane. According to one embodiment, said first and second nozzles are arranged at the same radial distance from said central axis.

According to one embodiment, each said first and second duct has in sequence a first cylindrical portion, a trapezoidal narrowing and a substantially cylindrical terminal section inclined with respect to said first plane. According to one embodiment, each said first and second duct has an intermediate section arranged between said narrowing and said terminal section.

According to one embodiment, each said terminal section is parallel to said second plane.

According to one embodiment, each said terminal section is inclined with respect to said second plane.

According to one embodiment, said top is faceted and has a central flat portion transversal to said axis and four faces that extend from said flat portion around said axis; two first faces of said four faces having the same shape and extension, in addition to being arranged symmetrically with respect to said median plane, and two faces of said four faces having equal shape and extension, in addition to being arranged symmetrically with respect to said second median plane.

According to one embodiment, each of said first and second nozzles are obtained in each of said second faces at the back of a respective outer edge.

According to one embodiment, said upper portion is engaged by a third duct concentric with said central axis; said third duct ending with a third nozzle obtained in said central flat portion.

According to one embodiment, said third duct extends inside said lower portion; said first, second and third ducts extend from said base portion to said top.

According to one embodiment, said upper portion has a cylindrical portion concentric with said axis and contained inside said lower portion to define with the latter an axial cylindrical guide for an end portion of a cylindrical supply duct of said liquid.

According to one embodiment, said cylindrical portion has an outer shell provided with two longitudinal grooves coaxial with said first and second ducts to define, in use, a supply channel for each said first and second ducts when said end portion of said cylindrical duct couples with said cylindrical guide.

According to one embodiment, said lower portion is hollow and is provided with a central chamber concentric with said axis.

According to one embodiment, said lower portion is hollow and is provided with a common central chamber for supplying said first, second and third ducts.

According to one embodiment, a dispensing set is provided comprising a bag containing medical liquid and provided with a delivery outlet for said liquid; a flexible duct being connected to said delivery outlet in a fluid-tight manner; characterized in that said flexible duct has one end connected to a dispenser as described above.

### BRIEF DESCRIPTION OF THE FIGURES

The invention will be better described with reference to some non-limiting embodiments in the attached figures, in which:
- figure A is a schematic view of a set which comprises a dispenser according to the present invention;
- figure B is a schematic view of the set of figure A in a preferred mode of use;
- figure 1 is a plan view of a first preferred embodiment of a dispenser according to the present invention;
- figure 2 is a view from below of figure 1;
- figure 2a illustrates a diagram referred to in the description with reference to the use of figure 1;
- figure 3 is a lateral elevation view of figure 1;
- figure 4 is a frontal elevation view of figure 1;
- figure 5 is a sectional view according to the line V-V of figure 3;
- figure 6 is a schematic perspective view from below of figure 5;
- figure 7 is a sectional view according to the line VII - VII of figure 2;
- figure 8 is a schematic perspective view from below of figure 7;
- figure 9 is a sectional view according to the line IX - IX of figure 4;
- figure 10 is a schematic perspective view from below of figure 9;
- figure 11 is a sectional view according to the line XI - XI of figure 4;
- figure 12 is a schematic perspective view from below of figure 11;
- figure 13 is a view from below of a second preferred embodiment of figure 1;
- figure 14 is a sectional view according to the line XIV - XIV of figure 13;
- figure 15 is a schematic perspective view from below of figure 14;
- figure 16 is a sectional view according to the line XVI - XVI of figure 13;
- figure 17 is a schematic perspective view from below of figure 16;
- figure 18 is a sectional view according to the line XVIII - XVIII of figure 13;
- figure 19 is a schematic perspective view from below of figure 17;
- figure 20 is a plan view from below of a third preferred embodiment of figure 1;

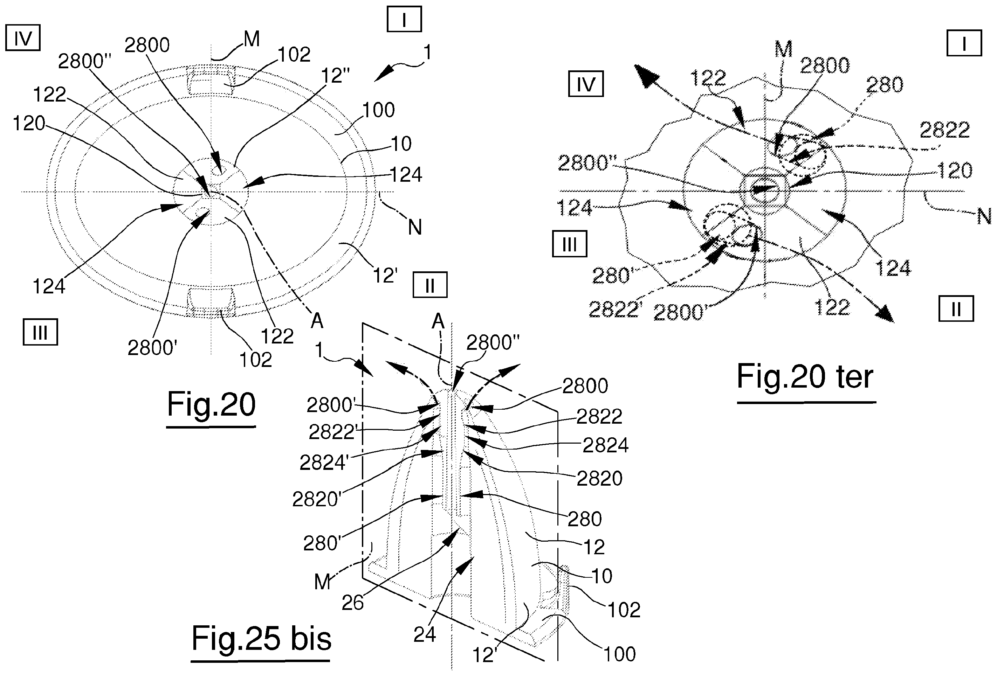

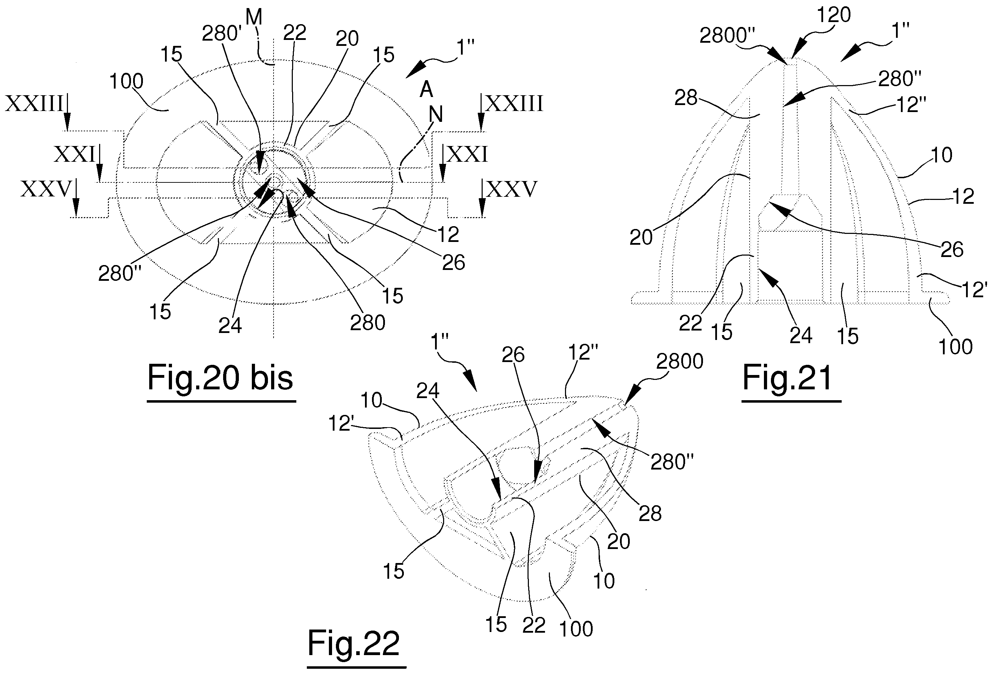

- figure 20bis is a view from below of figure 20;
- figure 20ter is a view on an enlarged scale of a detail of figure 20 with hidden lines exposed to view;
- figure 21 is a sectional view according to the median line XXI - XXI of figure 20;
- figure 22 is a schematic perspective view from below of figure 21;

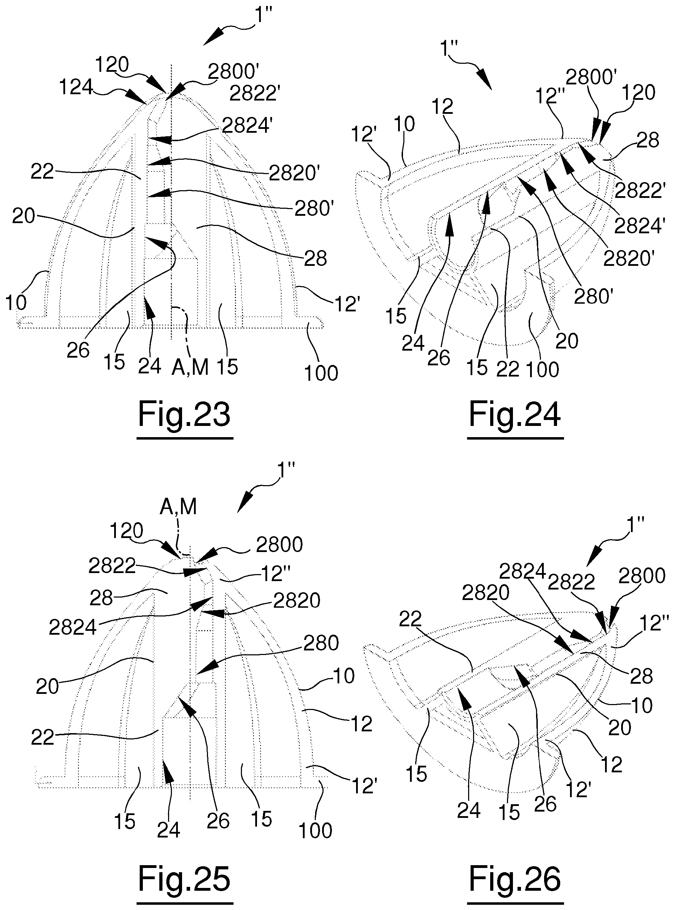

- figure 23 is a sectional view according to the median line XXIII - XXII of figure 20;
- figure 24 is a schematic perspective view from below of figure 23;
- figure 25 is a sectional view according to the median line XXV - XXV of figure 20;
- figure 25bis is a sectional view of figure 20 according to a section plane centred on the central axis and rotated with respect to two median planes of figure 20;
- figure 26 is a schematic perspective view from below of figure 23;

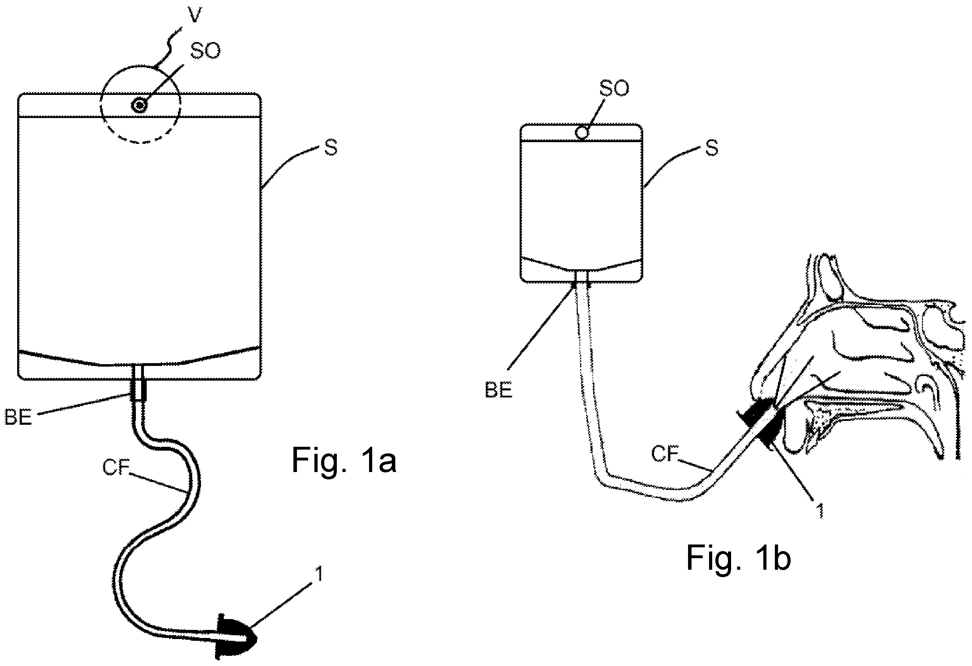

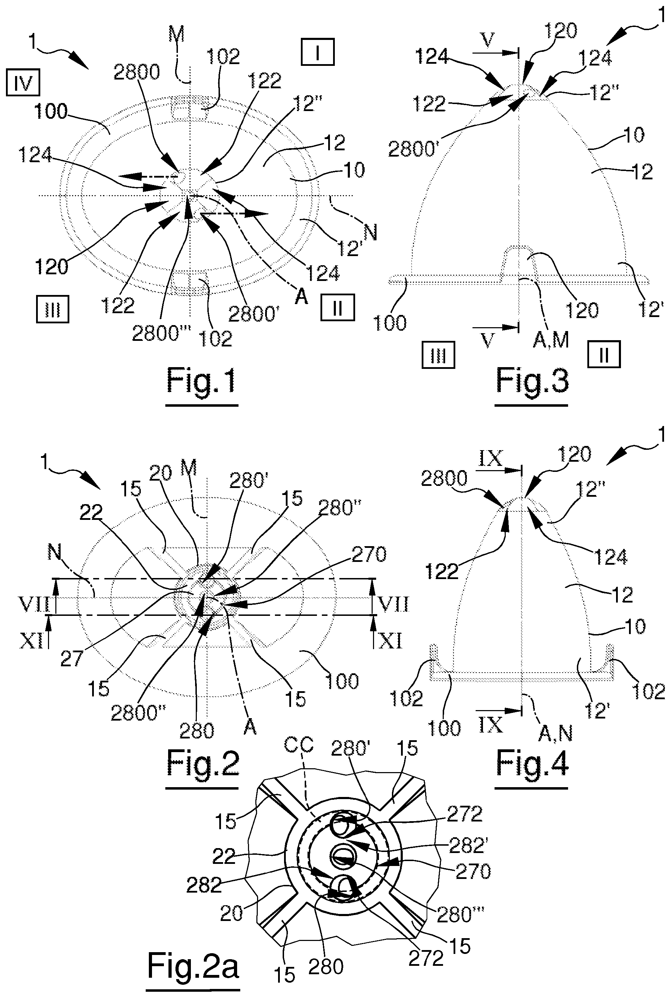

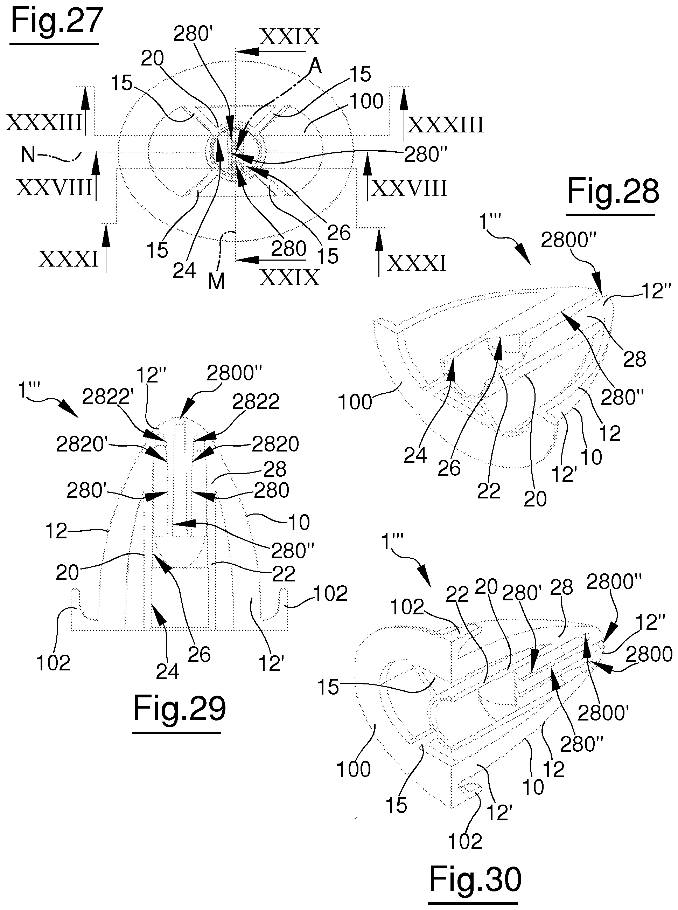

- figure 27 is a view from below of a fourth preferred embodiment of figure 1;
- figure 28 is a perspective sectional view according to the median line XXVIII - XXVIII of figure 27;
- figure 29 is a sectional view according to the median line XXIX - XXIX of figure 27;
- figure 30 is a schematic perspective view from below of figure 29;

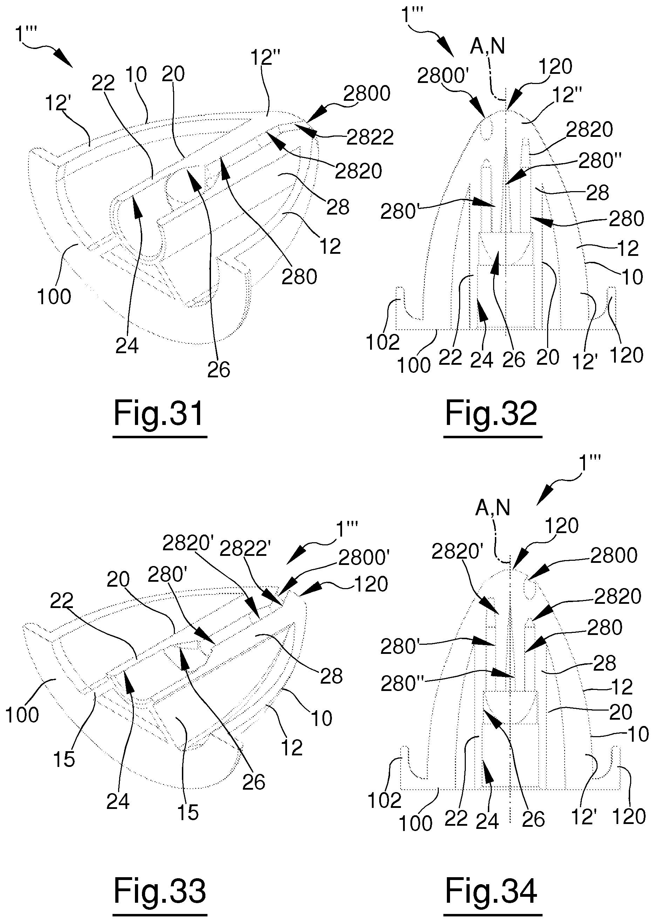

- figure 31 is a sectional view according to the median line XXXI - XXXI of figure 27;
- figure 32 is a symmetrical sectional view according to a first plane parallel to the line XXIX of figure 27;
- figure 33 is a sectional view according to the median line XXXIII - XXXIII of figure 27;
- figure 34 is a symmetrical sectional view of figure 32 according to a second plane parallel to the line XXIX of figure 27;

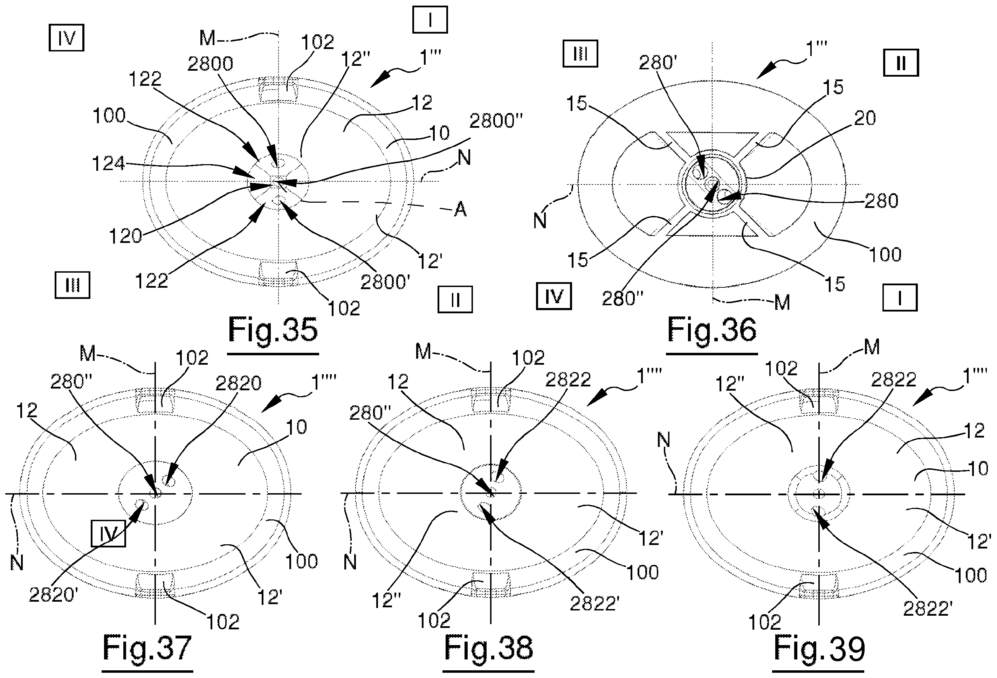

- figure 35 is a plan view of a fifth preferred embodiment of figure 1;
- figure 36 is a view from below of figure 35;
- figures 37-39 are cross-sectionals of figure 1 with respect to a central axis of increasing size starting from the base portion;

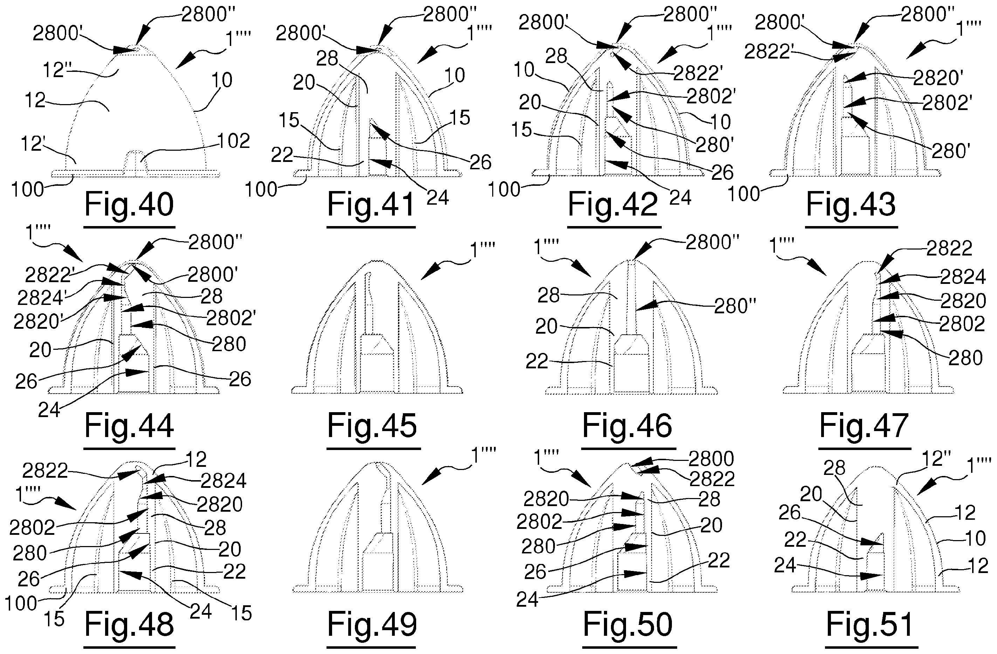

- figure 40 is a lateral elevation view of figure 35; and
- figures 41-51 are sections of figure 40 executed with a plurality of planes parallel to that of the sheet.

### DETAILED DISCLOSURE OF THE PRESENT INVENTION

In figure 1, the reference number 1 indicates a liquid dispenser 1 for medical washes. According to figures A and B, the liquid is contained in a bag S connected to the dispenser 1 by a small-diameter flexible tube CF in fluid-tight hydraulic communication with the bag S through a delivery outlet BE obtained peripherally in said bag S, and in particular at the bottom in figure A. The latter furthermore has an eyelet SO on the opposite side to the delivery outlet BE which can be used to hang the bag S on a wall beside a sanitary fitting (a wash basin or other as required) in an elevated position with respect to the area to be sprayed with liquid to control the outlet thereof from the bag S through the tube CF, and therefore the dispenser 1, exclusively by means of gravity. By way of non-limiting example, the bag S can be hung through the eyelet SO on a hook carried by a suction pad V applied to a smooth surface like that of a tile or a mirror and, due to the difference in height between the bag S and the dispenser 1, the liquid can supply the dispenser 1 after flowing from the bag S through the flexible tube CF.

The dispenser 1 comprises a first membrane body 10 shaped symmetrically with respect to a first frontal median plane M and a second sagittal median plane N which identify in plan four quadrants I, II, III, IV, in a similar manner to the Cartesian plane of analytical geometry. The indications of these quadrants are shown in boxes in figures 1 and 2. The first frontal and second sagittal median planes M and N are intersected in a central axis A, vertical in figure 1.

To each of the lines of the median planes M and N corresponds an axis of symmetry for the sections transversal to the central axis of the same first body 10, so that overall the dispenser 1 has a geometric conformation compatible with that of the nostrils, therefore suitable to be easily inserted in both and contained by them in a fluid-tight manner. Without conditioning the scope of the present invention, the first membrane body 10 can be made of pressure-deformable plastic material, for example, but not limited to, PVC, silicone or any other material having equivalent mechanical characteristics, or can be made of rigid plastic material, for example, but not limited to, ABS.

With particular reference to figures 1-4, the first body 10 comprises a substantially dome-shaped portion 12, thus named due to the shape of the respective outer surface, and below referred to simply as dome 12. The latter extends from an annular flat body which here and below will be indicated by the term crown and indicated by the reference number 100.

The crown 100 is provided with a pair of projections 102 symmetrically opposed with respect to the first frontal median plane M and oriented substantially parallel to the second sagittal median plane N. The presence of these projections 12 is optional for implementation of the dispenser 1 according to the following description.

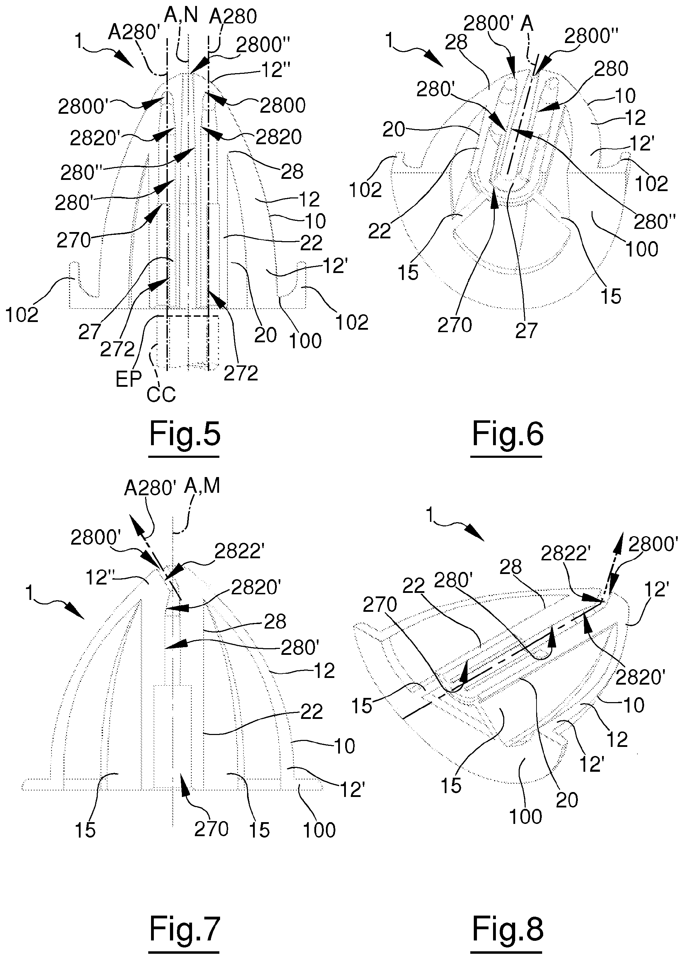

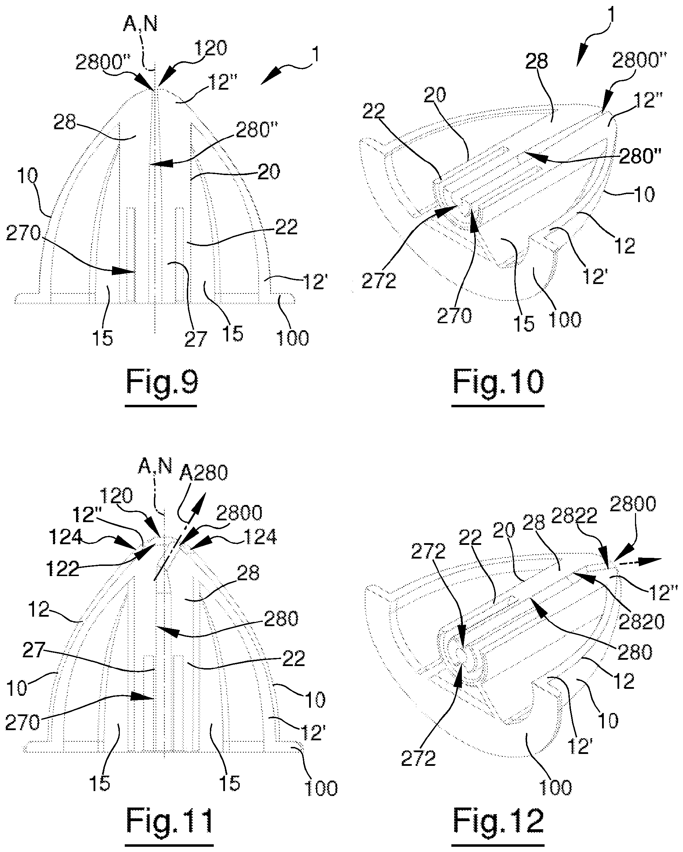

The dome 12 is axially delimited by a base portion 12', in turn delimited by the crown 100, and by a substantially faceted convex apical portion/top 12'' which has a central flat portion 120 transversal to the axis A and four faces that extend from the flat portion 120 around the axis A transversally to the first frontal and second sagittal median planes M and N. Of these four faces, two first faces 122 are opposed, having the same shape and extension, in addition to being arranged symmetrically with respect to the median plane M; two second faces 124 are opposed, having the same shape and extension, in addition to being arranged symmetrically with respect to the second median plane N. With particular reference to figures 5-12, the dispenser comprises a second body 20 carried by the first body 10 inside the dome 12 in a position concentric to the axis A. The connection between the first body 10 and the second body 20 is stiffened by substantially radial ribs 15.

The second body 20 has a hollow lower portion 22 which is also concentric with the axis A and an upper portion 28 connected to the dome 12. With particular reference to fig. 5, 6, 10, 12, the upper portion 28 has at the bottom a cylindrical portion 27 contained inside the lower portion 22 and concentric with the axis A to define with the latter an axial cylindrical guide 270 for an end portion EP of a cylindrical tube CC supplying the liquid to the dispenser 1, only partially visible in figure 5 where it is shown by a broken line. Said guide 270 requires the end portion EP of the cylindrical tube CC that supplies the dispenser 1 with the washing liquid to be inserted by pressure, avoiding the use of gluing solutions such as, for example, but not limited to, cyclohexanone to stabilize the reciprocal connection.

The lower portion 22 and upper portion 28 are engaged by a first duct 280 and by a second duct 280' terminating in the same dome 12 with a first nozzle 2800 and a second nozzle 2800' respectively where the first nozzle 2800 and the second nozzle 2800' are respectively arranged between the first frontal median plane M and the second sagittal median plane N at the same radial distance from the central axis A and on planes parallel to the second sagittal median plane N, therefore in a similar manner to the Cartesian plane in the II and IV quadrants in figure 1.

Furthermore, the first and the second duct 280/280' have respective longitudinal axes A280/A280' which are parallel to the central axis A in addition to being arranged at substantially identical distances from said central axis A; they lie on the plane M throughout the part contained in the lower portion 22 and the first part of the upper portion 28, while the end parts of the first and second ducts 280/280' are inclined with respect to the first frontal median plane M but lie on planes parallel to the second sagittal median plane N, opening into the respective first and second nozzles 2800/2800' (figures 5-8). In particular, the first and second ducts 280/280' have respectively a narrowing 2820/2820' upstream of the respective first/second nozzle 2800/2800', the longitudinal section has a substantially rectangular trapezium shape, continuing with an inclined cylindrical terminal section 2822/2822' which terminates in the nozzle 2800/2800', the outlet direction of which is parallel to the second sagittal median plane N and at least one component transversal to the first frontal median plane M, as in the arrows shown in figures 1, 7, 8, 11, 12.

Further to the above description, the first and second ducts 280/280' have respective terminal sections 2822/2822' that terminate in the first and second nozzles 2800/2800' with outlet direction (shown by a dash-dot line terminating in an arrow in figure 1) which, moving away from the first frontal median plane M is distanced point by point from the second sagittal median plane N by a length greater than or equal to the distance of the corresponding first and second nozzles 2800/2800' from said second sagittal median plane N. It can be easily understood that the outlet direction is parallel to the plane N in the case of the outlet direction from the terminal sections 2822 and 2822' being distanced point by point from the second sagittal median plane N by a length equal to the distance of the corresponding first and second nozzles 2800/2800' from said second sagittal median plane N.

Again further to the above description, considering that the two terminal sections 2822 and 2822' exit from the dome 12 with opposite directions, and that each corresponding jet is emitted by gravity from the nozzles 2800 and 2800', each jet moves away from the first frontal median plane M/central axis A shaped in a substantially cylindrical manner and, in any case, determined by the cross section of the terminal section 2822/2822' and by the shape of the nozzle 2800/2800', while keeping a distance from the second sagittal median plane N, and in particular parallel to the latter, also when the pressure drops due to the progressive decrease in the liquid column contained in the bag S, therefore independently of the current value of the gravity acting on the liquid in the bag S.

Each of the first and second nozzles 2800/2800' is obtained in one of the second faces 124 at the back of a respective outer edge, so that they are inclined with respect to the first frontal median plane M, from which they move away towards the top 12". The terminal sections 2822 and 2822' of the first duct 280 and second duct 280' are inclined with respect to the first frontal median plane M but lie on planes parallel to the second sagittal median plane N, so that the jets delivered by the first and second nozzles 2800/2800' remain parallel to the second sagittal median plane N and are inclined with respect to the first frontal median plane M, from which said jets move away (figures 1, 7, 8, 11, 12). The version of the dispenser 1 provided with terminal sections 2822 and 2822' which terminate curved in the first and second nozzles 2800/2800' also falls within the present invention.

Again with reference to figures 1-12, the lower portion 22 and the upper portion 28 are engaged by a third duct 280", which is concentric with the central axis A, has a tapered conical shape proceeding from the base portion 12' to the top 12'' and terminates in a third nozzle 2800'' which, therefore, is obtained centrally in the central flat portion 120. Furthermore, the third duct 280'' extends also inside the lower portion 22.

The cylindrical portion 27 has an outer shell 271 provided with two longitudinal grooves 272 coaxial with the first and second ducts 280/280' to define, in use, with the cylindrical body CC installed (an outline of which is visible only in figure 2a), a supply channel 282/282' for each first and second ducts 280/280' when the end portion EP of the cylindrical duct CC couples with the cylindrical guide 270. Said supply channels 282/282', which are formed in use once the end portion EP of the cylindrical duct CC is installed inside the guide 270, have a section transversal to the axis A having reduced form with respect to the cylindrical transversal section of the first and second ducts 280/280', since an external portion of the end portion EP of the cylindrical duct CC must be subtracted from the circular section of said ducts, as illustrated in figure 2A. In this figure the external portions of the first and second ducts 280/280' to be subtracted from the inside of the lower portion 22, as they cannot be engaged by the liquid, are shown by lines slanting at 45°.

The use of the dispenser 1 can be easily understood from the above description and does not require further explanation. However, it may be useful to specify that the first body 10 is shaped to easily treat the nasal cavities; in fact, the dome 12 is shaped to be inserted at least partially inside a nostril to be treated; in this way the external wall of the dome 12 adheres to the internal walls of the nostril, widening them so that the first and the second nozzles 2800 and 2800' (of the first and second ducts 280 and 280') and the third nozzle 2800" are positioned at the inlet of the nasal cavity. With the dome 12 of the dispenser 1 held firm in the position described above, said dispenser 1 can deliver medical liquid in the form of substantially cylindrical jets with filiform dimension. The experimental tests carried out showed that the lateral jets and the central jet remain constantly separate throughout the washing phase and that this situation remains unchanged even when the pressure of the washing liquid drops, considering that the pressure of the liquid is produced exclusively by the force of gravity and therefore by the level difference between the free surface of the liquid contained in the tank and the nozzles described above of the dispenser 1. Without limiting the scope of the present invention, the medical liquid contains physiological solution.

It may also be useful to specify that, further to the above description, considering that a connection portion of the first and second ducts 280 and 280' is contained in the lower portion 22 of the second body 20 and has reduced section when the guide 270 is engaged by the cylindrical body CC, in use, the liquid delivered to the first and second ducts 280 and 280' undergoes, in use, an expansion upstream of the respective first and second nozzles 2800/2800' and downstream of the portion of the first and second ducts 280/280' contained in the lower portion 22 of the second body 20.

Lastly it is clear that variations can be made to the dispenser 1 described and illustrated above without departing from the scope of the present invention.

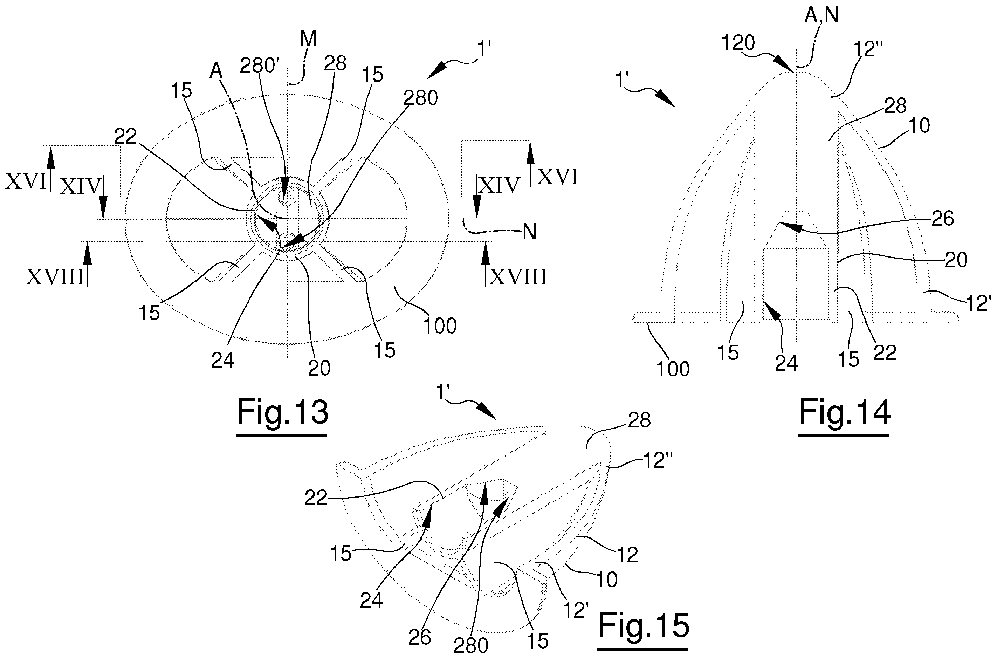

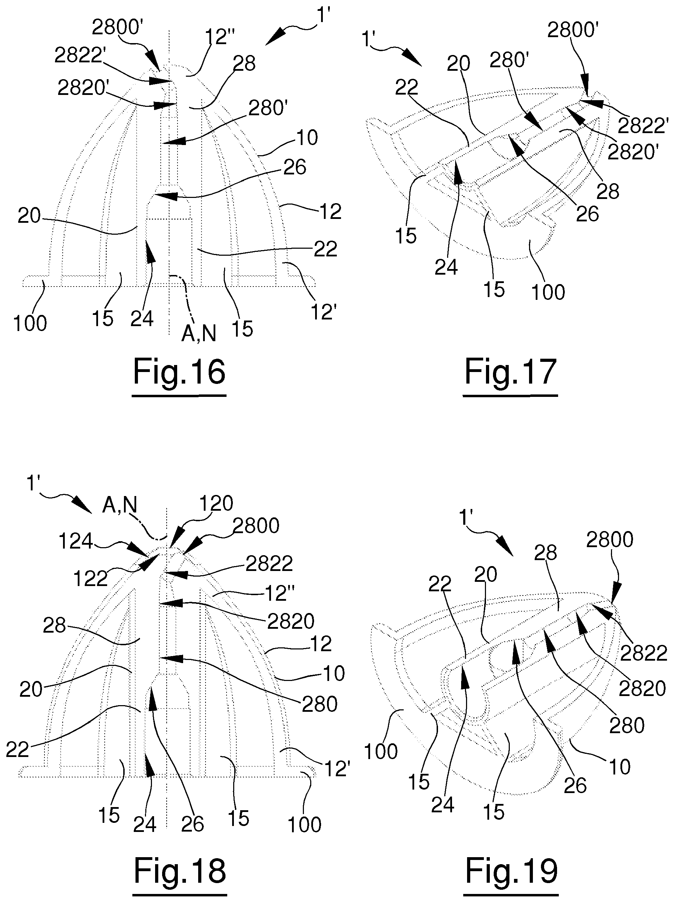

For example, with reference to figures 13-19 a variation of the dispenser 1 is illustrated, where homologous structural details will be indicated for the sake of practicality by the same reference numbers as those used in figures 1-12. The dispenser 1' is functionally and aesthetically similar to the dispenser 1 but is provided exclusively with the first and second ducts 280 and 280', arranged identically to the version of figures 1-12.

The dispenser 1' differs from the dispenser 1 described above due to the fact that the lower portion 22 is provided with a cylindrical seat 24 which is concentric to the axis A and is delimited at the top by a cylindrical portion with reduced diameter which is at the base of a tapered portion 26 clearly visible in figures 14-19. The cylindrical portion 27 is therefore lacking and the cylindrical base of the tapered portion 26 acts as an abutment for a hollow cylindrical body which can be inserted inside the cylindrical seat 24 to supply the liquid to the first and second ducts 280/280' of the dispenser 1'. The description of the other components shared with the first version of figures 1-12 is omitted for the sake of textual economy, but are indicated in figures 13-19 for the sake of practicality and to facilitate understanding of said embodiment of the invention.

It is therefore evident that in said case the jets emitted from the first and second nozzles 2800/2800' of figures 13-19 behave exactly like the jets emitted from the first and second nozzles 2800 and 2800' of figures 1-12 and, therefore, are diffused to the outside of the dome 12 parallel to the second sagittal median plane N of the dispenser 1' and thus remain spaced from one another and from the second sagittal median plane N moving away from the top 12'' of the first body 10.

With reference to figures 20-26 a dispenser 1" is illustrated which is a further variation of the dispenser 1. This variation incorporates some of the characteristics of the dispenser 1 of figures 1-12, since there are three ducts and nozzles, but it has a lower portion 22 which is substantially identical to that of figures 13-19. It is useful to point out that figure 20 ter is schematic and has the sole purpose of showing in plan a projection of the various parts composing the first and the second ducts 280/280' to facilitate understanding of the respective conformation which will be described better below.

In particular, in the lower portion 22 and approximately as far as the first half of the upper portion 28, the axes of the first, second and third ducts 280, 280', 280" lie arranged on a plane centred on the central axis A but inclined both with respect to the first frontal median plane M and with respect to the second sagittal median plane N, as can be clearly seen in figure 20 bis.

Furthermore, considering the upper half of the upper portion 28, the first and the second ducts 280/280' remain parallel to the axis A and have a section with decreasing section for approximately one third of said part of the upper portion 28. Said section with decreasing section is analogous to the narrowing 2820/2820' of figures 7, 8, 11 and 12, hence it is indicated by the same reference number. Going towards the top 12'', for the second third of the upper portion 28 in question, the first and the second ducts 280/280' remain parallel to the axis A and have respective sections 2824/2824' with substantially cylindrical and constant cross section; lastly, for the remaining third of the apical part of the upper portion 28, which leads to the first and second nozzles 2800 and 2800', the first and the second ducts 280/280' have inclined cylindrical sections similar to the terminal sections 2822/2822' of figures 7, 8, 11, 12. It may be useful to specify that these terminal sections 2822 and 2822' have a substantially circular and constant cross section and that, in use, the jets delivered from the first and second nozzles 2800 and 2800' of figures 20-26 cross the first frontal median plane M remaining substantially cylindrical and filiform, moving away, and therefore diverging, from the second sagittal median plane N and, naturally, from the axis A.

It should be noted that the terminal sections 2822 and 2822' of the first and second ducts 280/280' of this embodiment of the present invention proceed towards the top 12" according to divergent outlet directions, visible in figures 20ter and 25bis where they are shown by dash-dot lines terminating in arrows, where said arrows indicate the diffusion direction of the jets of liquid delivered by the first and second nozzles 2800 and 2800'. Further to the above description, also in this case the first and second ducts 280/280' have respective terminal sections 2822/2822' which terminate in the first and second nozzles 2800/2800' with outlet direction which, moving away from the first frontal median plane M, and therefore also from the central axis A and from the top 12", is distanced point by point from the second sagittal median plane N by a length greater than or equal to the distance of the corresponding first and second nozzles 2800/2800' from said second sagittal median plane N.

In particular, it is useful to specify that terminal sections 2822 and 2822' can be constructed either curved or rectilinear without modifying the scope of the present invention or producing a different result in terms of divergence of the jets emitted from the respective first and second nozzles 2800 and 2800'.

With reference to figures 27-34 a dispenser 1‴ is illustrated, which constitutes a further variation of the present invention. The dispenser 1‴ combines the upper portion 28 of the dispenser 1 of the present invention with the lower portion 22 of the dispenser 1' and the dispenser 1", since here again the lower portion 22 is hollow inside and has a cylindrical seat which terminates at the top in the tapered portion 26, already seen in figures 14-26. Therefore, to better understand the conformation of the dome 12 of said version of dispenser 1‴, reference should be made to figure 1 for the sake of economy of drawing. Unlike the version of figures 13-19, in this case the upper portion 28 also has the central third duct 280'', which terminates concentrically on the central axis A with the respective central third nozzle 2800" and unlike figures 20-26 the first and second ducts 280 and 280' have their respective inlets aligned with and centred on the first median plane M and arranged symmetrically with respect to the second sagittal median plane N. In addition, it should be noted that, as can be seen in figures 29-34, proceeding from the crown 100 towards the top 12'', the first and second ducts 280/280' have a first cylindrical section 2802/2802' substantially parallel to the axis A, followed by a narrowing 2820/2020' with substantially trapezoidal longitudinal section, developed around an axis substantially parallel to the central axis A. Said narrowing 2820/2020' is followed by a substantially cylindrical terminal section 2822/2822' (figure 31 for the section 2822 and figure 33 for the section 2822') and inclined with respect to the first frontal median plane M and second sagittal median plane N to terminate centred in the first/second nozzle 2800/2800'.

With particular reference to figures 35 - 51, a dispenser 1ʺʺ is illustrated which constitutes a further variation of the present invention. In the dispenser 1ʺʺ the inlets of the first, second and third ducts 280, 280' and 280", which open into the lower portion 22, are arranged aligned on a plane which is concentric to the central axis A and inclined with respect to the first and second sagittal median planes M and N; therefore, said plane cuts the quadrants I and III substantially at 45° as in figures 20-26. Unlike the dispenser 1‴, the first and second nozzles 2800, 2800' symmetrically engage the first two faces 122 of the top 12", being cut symmetrically by the first frontal median plane M and arranged on opposite sides, and at the same distance with respect to the second sagittal median plane N, while the third nozzle 2800" is concentric with the central axis A, being identical to the third duct 280" of the dispensers 1" and 1‴.

In addition, the first and second ducts 280 and 280' are identical, obtained inside the upper portion 28 and are rotated with respect to each other by 180° so that they are polarly symmetrical with respect to the central axis A. In particular, as can be seen in figures 37-38, which are sections of the dispenser 1 arranged transversally to the axis A and at increasing distances starting from the crown 100, the first and second ducts 280 and 280' have a sequence of sections with form similar to the form of the homologous sections of the ducts 280 and 280' of the dispenser 1" (figures 20-26) although the respective axes have a portion inclined towards the top 12'' of the dispenser to supply the nozzles 2800 and 2800' arranged symmetrically to the first frontal median plane M and on opposite equally distant sides from the second sagittal median plane N. The same trend of the first and second ducts 280 and 280' can be seen in figures 41-51, each of which is a section of the dispenser 1ʺʺ obtained with respect to a plane parallel to the second sagittal median plane N, starting from a plane more external with respect to the section of figure 46 to terminate in a more internal plane. In particular, as can be seen in figures 42-50, proceeding from the crown 100 towards the top 12'' the first and second ducts 280/280' of the dispenser 1ʺʺ have a first cylindrical section 2802/2802', followed by a narrowing 2820/2020' with substantially trapezoidal longitudinal section, developed around an axis substantially parallel to the central axis A. Proceeding again towards the top 12'', said narrowing 2820/2020' is followed by an intermediate substantially cylindrical section 2824/2824' the axis of which is parallel to the central axis A but eccentric with respect to the axis of the first cylindrical section of the duct 280/280'. Each intermediate section 2824/2824' is followed by a substantially cylindrical terminal section 2822/2822' (figures 42-44 for the section 2822' and figures 48-50 for the section 2822') inclined with respect to the first frontal median plane M and the second sagittal median plane N to terminate centred in the first/second nozzle 2800/2800'.

Further to the above description, it can be understood that a dispenser shaped like any one of the dispensers 1, 1', 1", 1‴ 1ʺʺ, therefore provided with two lateral nozzles 2800 and 2800' arranged on opposite sides of a median plane and, possibly, of a central nozzle 2800‴ inserted in a set comprising a bag S containing medical liquid and a flexible tube CF that connects an outlet BE of the bag S to the dispenser 1 by the sole action of the gravitational force acting on the liquid column, allows, in use, the delivery of two or three jets which remain separate as they move away from the top 12" of said dispenser 1, 1', 1", 1‴ 1ʺʺ, allowing integral spraying of the mucosa that covers the entire nasal cavity even when the level of the free surface of the liquid contained in the tank S, and therefore the pressure acting on the washing liquid, drops.

## Claims

1. A gravity feed dispenser (1) of liquid for medical uses wherein a first membrane body (10) has at the top a dome (12) axially delimited by a base portion (12') and by a top (12") of given shape provided with a central axis positioned at the intersection of a first frontal median plane (M) and a second sagittal median plane (N); a second body (20) being carried internally by said first body (10); said second body (20) having a hollow lower portion (22) concentric with said central axis (A) and an upper portion (28) engaged by a first duct (280) and by a second duct (280'), both terminating in said dome (12), with a first nozzle (2800) and a second nozzle (2800') respectively; wherein said first and second ducts (280) (280') have respective terminal sections (2822) (2822') which terminate in said first and second nozzles (2800) (2800') with outlet direction which, moving away from said first frontal median plane (M), is distanced point by point from said second sagittal median plane (N) by a length greater than the distance of the corresponding said first and second nozzles (2800) (2800') from said second sagittal median plane (N), **characterized in that** each said first and second duct (280) (280') have in sequence a first cylindrical portion (2802) (2802'), a trapezoidal narrowing (2820) (2820') and a substantially cylindrical terminal section (2822) (2822') inclined with respect to said first frontal median plane (M), wherein the delivery pressure of the liquid is determined exclusively by gravity.

2. The dispenser according to claim 1, **characterized in that** said first and second nozzles (2800) (2800') are arranged at the same radial distance from said central axis (A).

3. The dispenser according to claim 1 or 2, **characterized in that** each said first and second duct (280) (280') has an intermediate section (2824) (2824') arranged between said narrowing (2820) (2820') and said terminal section (2822) (2822').

4. The dispenser according to claim 3, **characterized in that** each said terminal section (2822) (2822') is parallel to said second sagittal median plane (N).

5. The dispenser according to claim 3, **characterized in that** each said terminal section (2822) (2822') is inclined with respect to said second sagittal median plane (N).

6. The dispenser according to any one of the preceding claims, **characterized in that** said top (12") is faceted and has a central flat portion (120) transversal to said axis (A) and four faces (122) (124) which extend from said flat portion (120) around said axis (A); two first faces (122) of said four faces (122) (124) having the same shape and extension, in addition to being arranged symmetrically with respect to said first frontal median plane (M) and two faces (124) of said four faces (122) (124) having equal shape and extension, in addition to being arranged symmetrically with respect to said second sagittal median plane (N).

7. The dispenser according to claim 6, **characterized in that** each of said first and second nozzles (2800) (2800') are obtained in each of said second faces (124) at the back of a respective outer edge.

8. The dispenser according to any one of the preceding claims, **characterized in that** said upper portion (28) is engaged by a third duct (280") concentric with said central axis (A); said third duct (280'') terminating in a third nozzle (2800") obtained in said central flat portion (120).

9. The dispenser according to any one of the preceding claims, **characterized in that** said third duct (280") extends inside said lower portion (22); said first, second and third ducts (280) (280') (280") extend from said base portion (12') to said top (12").

10. The dispenser according to claim 9, **characterized in that** said upper portion (28) carries a cylindrical portion (27) concentric with said axis (A) and contained inside said lower portion (22) to define with the latter an axial cylindrical guide (270) for an end portion (EP) of a cylindrical supply duct (CC) of said liquid.

11. The dispenser according to claim 10, **characterized in that** said cylindrical portion (27) has an outer shell (270) provided with two longitudinal grooves (272) coaxial with said first and second ducts (280) (280') to define, in use, a supply channel (282) (282') for each said first and second ducts (280) (280') when said end portion (EP) of said cylindrical duct (CC) couples with said cylindrical guide (270).

12. The dispenser according to any one of the claims 1-8, **characterized in that** said lower portion (22) is hollow and is provided with a central chamber (220) concentric with said axis (A).

13. The dispenser according to any one of the claims 1-8, **characterized in that** said lower portion (22) is hollow and is provided with a common central chamber (220') for feeding said first, second and third ducts (280) (280') (280").

14. A dispensing set comprising a bag (S) for medical liquid and provided with a dispensing outlet (BE) for said liquid; a flexible tube (CF) being connected to said dispensing outlet (BE) in a fluid-tight manner; **characterized in that** said flexible tube (CF) has one end (E) connected to a dispenser (1, 1', 1", 1‴, 1ʺʺ) according to any one of the preceding claims.

15. The set according to claim 14, **characterised in that** said bag contains said medical liquid.

## Patentansprüche

1. Ein schwerkraftgespeister Spender (1) von Flüssigkeit für medizinische Anwendungen, wobei ein erster Membrankörper (10) an der Oberseite eine Kuppel (12) aufweist, die axial durch einen Basisteil (12') und durch eine Oberseite (12") einer gegebenen Form begrenzt ist und mit einer Mittelachse versehen ist, die an der Schnittstelle einer ersten, frontalen Medianebene (M) und einer zweiten, sagittalen Medianebene (N) positioniert ist; wobei ein zweiter Körper (20) intern von dem ersten Körper (10) getragen wird; wobei der zweite Körper (20) einen hohlen unteren Teil (22), der mit der Mittelachse (A) konzentrisch ist, und einen oberen Teil (28) aufweist, die von einem ersten Durchgang (280) und von einem zweiten Durchgang (280') eingenommen werden, die beide mit einer ersten Düse (2800) bzw. einer zweiten Düse (2800') in der Kuppel (12) enden; wobei der erste und der zweite Durchgang (280) (280') jeweilige Endabschnitte (2822) (2822') aufweisen, die in der ersten und der zweiten Düse (2800) (2800') enden, mit einer Auslassrichtung, die mit zunehmender Entfernung von der ersten, frontalen Medianebene (M) Punkt für Punkt von der zweiten, sagittalen Medianebene (N) um eine Länge beabstandet ist, die größer als der Abstand der entsprechenden ersten und zweiten Düse (2800) (2800') von der zweiten, sagittalen Medianebene (N) ist, **dadurch gekennzeichnet, dass** jeder von dem ersten und dem zweiten Durchgang (280) (280') der Reihe nach einen ersten zylindrischen Teil (2802) (2802'), eine trapezförmige Verengung (2820) (2820') und einen im Wesentlichen zylindrischen Endabschnitt (2822) (2822'), der in Bezug auf die erste, frontale Medianebene (M) geneigt ist, aufweist, wobei der Abgabedruck der Flüssigkeit ausschließlich durch die Schwerkraft bestimmt wird.

2. Spender gemäß Anspruch 1, **dadurch gekennzeichnet, dass** die erste und die zweite Düse (2800) (2800') in demselben radialen Abstand von der Mittelachse (A) angeordnet sind.

3. Spender gemäß Anspruch 1 oder 2, **dadurch gekennzeichnet, dass** jeder von dem ersten und dem zweiten Durchgang (280) (280') einen Zwischenabschnitt (2824) (2824') aufweist, der zwischen der Verengung (2820) (2820') und dem Endabschnitt (2822) (2822') angeordnet ist.

4. Spender gemäß Anspruch 3, **dadurch gekennzeichnet, dass** jeder besagte Endabschnitt (2822) (2822') parallel zu der zweiten, sagittalen Medianebene (N) ist.

5. Spender gemäß Anspruch 3, **dadurch gekennzeichnet, dass** jeder besagte Endabschnitt (2822) (2822') in Bezug auf die zweite, sagittale Medianebene (N) geneigt ist.

6. Spender gemäß einem der vorhergehenden Ansprüche, **dadurch gekennzeichnet, dass** die Oberseite (12") facettiert ist und einen mittleren, flachen Teil (120) quer zu der Achse (A) und vier Flächen (122) (124), die sich von dem flachen Teil (120) um die Achse (A) erstrecken, aufweist; wobei zwei erste Flächen (122) der vier Flächen (122) (124) dieselbe Form und Ausdehnung aufweisen, zusätzlich dazu, dass sie in Bezug auf die erste, frontale Medianebene (M) symmetrisch angeordnet sind, und zwei Flächen (124) der vier Flächen (122) (124) eine gleiche Form und Ausdehnung aufweisen, zusätzlich dazu, dass sie in Bezug auf die zweite, sagittale Medianebene (N) symmetrisch angeordnet sind.

7. Spender gemäß Anspruch 6, **dadurch gekennzeichnet, dass** jede von der ersten und der zweiten Düse (2800) (2800') in jeder der zweiten Flächen (124) an der Rückseite einer jeweiligen Außenkante erhalten wird.

8. Spender gemäß einem der vorhergehenden Ansprüche, **dadurch gekennzeichnet, dass** der obere Teil (28) von einem dritten Durchgang (280") eingenommen wird, der mit der Mittelachse (A) konzentrisch ist; wobei der dritte Durchgang (280") in einer dritten Düse (2800") endet, die in dem mittleren, flachen Teil (120) erhalten wird.

9. Spender gemäß einem der vorhergehenden Ansprüche, **dadurch gekennzeichnet, dass** sich der dritte Durchgang (280") innerhalb des unteren Teils (22) erstreckt; wobei sich der erste, der zweite und der dritte Durchgang (280) (280') (280") von dem Basisteil (12') zu der Oberseite (12") erstrecken.

10. Spender gemäß Anspruch 9, **dadurch gekennzeichnet, dass** der obere Teil (28) einen zylindrischen Teil (27) trägt, der mit der Achse (A) konzentrisch ist und innerhalb des unteren Teils (22) enthalten ist, um mit dem letzteren eine axiale zylindrische Führung (270) für einen Endteil (EP) eines zylindrischen Zufuhrdurchgangs (CC) der Flüssigkeit zu definieren.

11. Spender gemäß Anspruch 10, **dadurch gekennzeichnet, dass** der zylindrische Teil (27) eine Außenhülle (270) aufweist, die mit zwei Längsrillen (272) versehen ist, welche koaxial mit dem ersten und dem zweiten Durchgang (280) (280') sind, um im Gebrauch einen Zufuhrkanal (282) (282') für jeden von dem ersten und dem zweiten Durchgang (280) (280') zu definieren, wenn der Endteil (EP) des zylindrischen Durchgangs (CC) mit der zylindrischen Führung (270) gekoppelt ist.

12. Spender gemäß einem der Ansprüche 1-8, **dadurch gekennzeichnet, dass** der untere Teil (22) hohl ist und mit einer mittleren Kammer (220) versehen ist, die mit der Achse (A) konzentrisch ist.

13. Spender gemäß einem der Ansprüche 1-8, **dadurch gekennzeichnet, dass** der untere Teil (22) hohl ist und mit einer gemeinsamen mittleren Kammer (220') zum Speisen des ersten, des zweiten und des dritten Durchgangs (280) (280') (280") versehen ist.

14. Ein Spendersatz, der Folgendes beinhaltet: einen Beutel (S) für medizinische Flüssigkeit und mit einem Spenderauslass (BE) für die Flüssigkeit versehen; einen flexiblen Schlauch (CF), der auf eine fluiddichte Weise mit dem Spenderauslass (BE) verbunden ist; **dadurch gekennzeichnet, dass** der flexible Schlauch (CF) ein Ende (E) aufweist, das mit einem Spender (1, 1', 1", 1‴, 1ʺʺ) gemäß einem der vorhergehenden Ansprüche verbunden ist.

15. Satz gemäß Anspruch 14, **dadurch gekennzeichnet, dass** der Beutel die medizinische Flüssigkeit enthält.

## Revendications

1. Un distributeur d'alimentation par gravité (1) de liquide pour des utilisations médicales dans lequel un premier corps de membrane (10) a au niveau du dessus un dôme (12) axialement délimité par une portion de base (12') et par un dessus (12") de forme donnée pourvu d'un axe central positionné à l'intersection d'un premier plan médian frontal (M) et d'un deuxième plan médian sagittal (N) ; un deuxième corps (20) étant porté intérieurement par ledit premier corps (10) ; ledit deuxième corps (20) ayant une portion inférieure creuse (22) concentrique avec ledit axe central (A) et une portion supérieure (28) mise en prise par un premier conduit (280) et par un deuxième conduit (280'), se terminant tous les deux par ledit dôme (12), avec une première buse (2800) et une deuxième buse (2800') respectivement ; dans lequel lesdits premier et deuxième conduits (280) (280') ont des sections terminales (2822) (2822') respectives qui se terminent par lesdites première et deuxième buses (2800) (2800') avec une direction de sortie qui, en s'écartant dudit premier plan médian frontal (M), est éloignée point par point dudit deuxième plan médian sagittal (N) d'une longueur supérieure à la distance desdites première et deuxième buses (2800) (2800') correspondantes dudit deuxième plan médian sagittal (N), **caractérisé en ce que** chaque dit premier et deuxième conduit (280) (280') a, en séquence, une première portion cylindrique (2802) (2802'), un rétrécissement trapézoïdal (2820) (2820') et une section terminale substantiellement cylindrique (2822) (2822') inclinée par rapport audit premier plan médian frontal (M), dans lequel la pression de délivrance du liquide est déterminée exclusivement par gravité.

2. Le distributeur selon la revendication 1, **caractérisé en ce que** lesdites première et deuxième buses (2800) (2800') sont agencées à la même distance radiale dudit axe central (A).

3. Le distributeur selon la revendication 1 ou la revendication 2, **caractérisé en ce que** chaque dit premier et deuxième conduit (280) (280') a une section intermédiaire (2824) (2824') agencée entre ledit rétrécissement (2820) (2820') et ladite section terminale (2822) (2822').

4. Le distributeur selon la revendication 3, **caractérisé en ce que** chaque dite section terminale (2822) (2822') est parallèle audit deuxième plan médian sagittal (N).

5. Le distributeur selon la revendication 3, **caractérisé en ce que** chaque dite section terminale (2822) (2822') est inclinée par rapport audit deuxième plan médian sagittal (N).

6. Le distributeur selon l'une quelconque des revendications précédentes, **caractérisé en ce que** ledit dessus (12") est à facettes et a une portion plate centrale (120) transversale audit axe (A) et quatre faces (122) (124) qui s'étendent à partir de ladite portion plate (120) autour dudit axe (A) ; deux premières faces (122) desdites quatre faces (122) (124) ayant les mêmes forme et extension, en plus d'être agencées symétriquement par rapport audit premier plan médian frontal (M) et deux faces (124) desdites quatre faces (122) (124) ayant des forme et extension égales, en plus d'être agencées symétriquement par rapport audit deuxième plan médian sagittal (N).

7. Le distributeur selon la revendication 6, **caractérisé en ce que** chacune desdites première et deuxième buses (2800) (2800') est obtenue dans chacune desdites deuxièmes faces (124) à l'arrière d'un bord externe respectif.

8. Le distributeur selon l'une quelconque des revendications précédentes, **caractérisé en ce que** ladite portion supérieure (28) est mise en prise par un troisième conduit (280") concentrique avec ledit axe central (A) ; ledit troisième conduit (280") se terminant par une troisième buse (2800") obtenue dans ladite portion plate centrale (120).

9. Le distributeur selon l'une quelconque des revendications précédentes, **caractérisé en ce que** ledit troisième conduit (280") s'étend à l'intérieur de ladite portion inférieure (22) ; lesdits premier, deuxième et troisième conduits (280) (280') (280") s'étendent de ladite portion de base (12') audit dessus (12").

10. Le distributeur selon la revendication 9, **caractérisé en ce que** ladite portion supérieure (28) porte une portion cylindrique (27) concentrique avec ledit axe (A) et contenue à l'intérieur de ladite portion inférieure (22) afin de définir avec cette dernière un guide cylindrique axial (270) pour une portion d'extrémité (EP) d'un conduit d'amenée cylindrique (CC) dudit liquide.

11. Le distributeur selon la revendication 10, **caractérisé en ce que** ladite portion cylindrique (27) a une coque externe (270) pourvue de deux rainures longitudinales (272) coaxiales avec lesdits premier et deuxième conduits (280) (280') afin de définir, en cours d'utilisation, un canal d'amenée (282) (282') pour chaque dit premier et deuxième conduit (280) (280') lorsque ladite portion d'extrémité (EP) dudit conduit cylindrique (CC) s'accouple audit guide cylindrique (270).

12. Le distributeur selon l'une quelconque des revendications 1 à 8, **caractérisé en ce que** ladite portion inférieure (22) est creuse et pourvue d'une chambre centrale (220) concentrique avec ledit axe (A).

13. Le distributeur selon l'une quelconque des revendications 1 à 8, **caractérisé en ce que** ladite portion inférieure (22) est creuse et est pourvue d'une chambre centrale commune (220') pour alimenter lesdits premier, deuxième et troisième conduits (280) (280') (280").

14. Un ensemble de distribution comprenant un sac (S) pour liquide médical et pourvu d'une sortie de distribution (BE) pour ledit liquide ; un tube flexible (CF) qui est raccordé à ladite sortie de distribution (BE) d'une manière étanche au fluide ; **caractérisé en ce que** ledit tube flexible (CF) a une extrémité (E) raccordée à un distributeur (1, 1', 1", 1‴, 1ʺʺ) selon l'une quelconque des revendications précédentes.

15. L'ensemble selon la revendication 14, **caractérisé en ce que** ledit sac contient ledit liquide médical.
